# EUROPEAN PATENT APPLICATION

(11) **EP 2 085 021 A1**
(43) Date of publication of application: **05.08.2009**
(21) Application number: 07806034.0
(22) Date of filing: 24.08.2007
(51) Int. Cl.: A61B 1/12, A61B 1/00, A61B 19/00, G02B 23/24

(54) **ENDOSCOPE CHANNEL INTERIOR CLEANING SYSTEM AND METHOD OF CLEANING ENDOSCOPE CHANNEL INTERIOR**

(30) Priority: 16.11.2006 JP 2006310802
(71) Applicant: KANNO, Minoru, Sendai-shi, Miyagi 980-0871 (JP)
(72) Inventor: NAKAYAMA, Takehisa, Shinjuku-ku Tokyo 169-0074 (JP)
(74) Representative: Jacob, Reuben Ellis
(86) International application number: PCT/JP2007/066427
(87) International publication number: WO 2008/059647

(57) **Abstract**

Provided are a system of cleaning the inside of an endoscope channel and a method of cleaning the inside of an endoscope channel, which allows to reduce a burden on an operator with enhanced safety with infection and contamination and a high cleaning effect. A first cleaning element 11 and a second cleaning element 12 are formed in a rotational symmetry with a given axis, provided with an external diameter larger than an inner diameter of a channel 1 of an endoscope and elastic in a direction perpendicular to the axis. The first cleaning element 11 is provided with a magnetic body. The first cleaning element 11 and the second cleaning element 12 are inserted into the inside of a channel 1 of an endoscope to conform the axial direction with a length direction of the channel 1. Cleaning water is injected into the channel 1 to move the first cleaning element 11 and the second cleaning element 12 inside the channel 1. A magnet of a guiding device 13 configured outside the channel 1 is acted on the magnetic body of the first cleaning element 11 to guide the first cleaning element to a branch channel 1b.

## Description

### TECHNICAL FIELD OF THE INVENTION

The present invention relates to a system of cleaning the inside of an endoscope channel and a method of cleaning the inside of an endoscope channel.

### BACKGROUND ART

A long stainless steel wire equipped with a brush at tip is generally used heretofore to insert into an opening in the side at hand of each channel to manually clean with the brush as a conventional method of cleaning the inside of a forceps channel, a suction channel and an air-supply and water-supply channel in an endoscope. There is also a method to use an automatic washing machine to drive a wire with a brush with a rubber roller instead of manual operation. (For example, see Patent Document 1).

Patent Document 1: Japanese Patent Publication 2003-10116

### PROBLEMS TO BE SOLVED BY THE INVENTION

A conventional cleaning of a wire with a brush can realize a high cleaning effect when cleaned carefully, but it is required to that end to repeat an operation, in which the brush inserted into a channel is stuck out through an opening at the tip of a channel to rub the brush with a finger in a cleaning liquid to remove contaminants remained and then pulled back to the opening in the side at hand to remove contaminants on the brush pulled out by similar operation, thus repeating insertion of the brush again to remove contaminants and then pullback.

Accordingly, there has been a problem, in which this procedure takes time and an operator to perform the cleaning has a heavier burden. There has been a problem, in which an operator directly washes a brush attached with contaminants with a finger, leading to a higher risk with infection even if a working glove is worn. There has been a problem, in which contaminants and pathogens remained on a root of bristles of the brush and a wire are likely to attach again the inner wall of a channel, since the brush and wire are inserted into the channel without removal thereof. Furthermore, there has been a problem, in which when the brush is pulled out from the opening, bristles of the brush compressed to the inner wall of the channel bounce to splash fine droplets containing contaminants so that cross-contamination is likely to occur with an operator for cleaning and a cleaning equipment and the operator of cleaning could be in danger of being infected depending on pathogens contained in the contaminated substance.

### OBJECTS OF THE INVENTION

The present invention has been performed in concerning with such problems and has a purpose to provide a system of cleaning the inside of an endoscope channel and a method of cleaning the inside of the endoscope channel, enabling to reduce a burden on an operator with enhanced safety with infection and contamination and a higher cleaning effect.

### SUMMARY OF THE INVENTION

In order to accomplish the purpose described above, a system of cleaning the inside of an endoscope channel associated with the present invention is one to clean the channel inside of an endoscope and comprises the features provided with a tool of cleaning the inside of an endoscope channel and a guiding device, in which the above endoscope is provided with a plurality of branch channels from the above channel, the above tool of cleaning the inside of an endoscope channel is formed in rotational symmetry with a given axis, provided with an external diameter larger than an inner diameter of the above channel in a cross-section perpendicular to the above axis in a given location, elastic in a direction perpendicular to the above axis and provided with a magnet or magnetic body and the above guiding device is provided with a magnet or magnetic body configured outside the above channel to act on the above magnet or magnetic body of the above tool of cleaning the inside of an endoscope channel to guide the above tool of cleaning the inside of an endoscope channel to one of the above branch channels.

A method of cleaning the inside of an endoscope channel associated with the present invention is one to clean the channel inside of an endoscope which is provided with a plurality of branch channels from the above channels, inserting the tool of cleaning the inside of an endoscope channel, which is formed in rotational symmetry with a given axis, provided with an external diameter larger than an inner diameter of the endoscope channel in a cross-section perpendicular to the above axis in a given location, elastic in a direction perpendicular to the above axis and provided with a magnet or magnetic body from a channel port of the above endoscope into the inside of the above channel from a channel port of the above endoscope to conform a direction of the above axis with a length direction of the above channel, injecting cleaning water through the above channel port to move the above tool of cleaning the inside of the endoscope channel inside the above channel and acting a magnet or magnetic body configured outside the above channel on the above magnet or magnetic body of the above tool of cleaning the inside of the endoscope channel to guide the above tool of cleaning the inside of an endoscope channel to one of the above branch channels.

In the system of cleaning the inside of an endoscope channel and the method of cleaning the inside of an endoscope channel associated with the present invention, the tool of cleaning the inside of an endoscope channel is inserted from the channel port of an endoscope into the inside of the channel to conform an axial direction with a length direction of the endoscope channel when used. The tool of cleaning the inside of an endoscope channel can be compressed in a direction perpendicular to the axis to easily insert into the channel inside, since it has an external diameter larger than an internal diameter of the channel in a cross-section perpendicular to the axis at a given location and is elastic in a direction perpendicular to the axis. Inserting this tool into the inside of a channel can lead to contact the inner wall surface of the channel. Cleaning water is injected in this state through the channel port to move the above tool along the channel inside with a water pressure thereof, enabling to remove contaminants and the like attached to the inner wall surface of the channel to clean the channel inside.

A burden on an operator can be thus reduced in the system of cleaning the inside of the endoscope channel and the method of cleaning the inside of the endoscope channel associated with the present invention as compared with a case of careful cleaning with a brush, since cleaning is performed by an operation, in which the tool of cleaning the inside of an endoscope channel is inserted into the channel inside followed by injecting cleaning water. Safety with infection and contamination is enhanced, since the operator does not directly contact the contaminants. The endoscope is placed in cleaning water in a cleaning vessel when operated, enabling to discharge the tool of cleaning the inside of an endoscope channel passed through the inside of the channel into cleaning water in the cleaning vessel. This can prevent droplets containing contaminants from splashing and re-contamination of the channel inside, resulting in enhanced safety with infection and contamination and a higher cleaning effect.

The tool of cleaning the inside of an endoscope channel may be formed in, for example, a cylindrical shape, spherical shape or elliptical shape with a length ranging from one to ten-fold of the inner diameter of the channel. This tool may be a brush, foam such as a polyurethane sponge or cellulose sponge, a rubber or elastic resin with a plurality of finny protrusions in a direction almost perpendicular to an axial direction, non-woven fabrics made from a resin or cellulosic fibers and the like and may be formed singly or in combination thereof. An abrasive material with hardness and surface shape not scratching the inner wall of the channel may be further attached to the surface thereof in order to improve the removal efficiency.

In the system of cleaning the inside of an endoscope channel and the method of cleaning the inside of an endoscope channel associated with the present invention, attractive force or repulsive force exerted between a magnet or magnetic body configured outside the channel and a magnet or magnetic body of the tool of cleaning the inside of an endoscope channel or attractive force exerted between the magnetic body configured outside the channel and the magnet of the tool of cleaning the inside of an endoscope channel can be utilized to guide the tool of cleaning the inside of an endoscope channel to any one of the branch channels to clean. For example, the magnet or magnetic body configured outside the channel is advanced to or distanced from a branching point of the channels, enabling to guide the tool of cleaning the inside of an endoscope channel. An electromagnet can be also configured outside the channel to control power distribution to the electromagnet guiding the tool of cleaning the inside of an endoscope channel. The magnet and ferromagnet in the tool of cleaning the inside of an endoscope channel is preferably made from a material of a ferrite type stainless steel, a martensite type stainless steel and the like in order to prevent from corrosion or dissolution with the cleaning liquid. It may also be cladded with a corrosion-resistant resin and the like.

In the system of cleaning the inside of an endoscope channel and the method of cleaning the inside of an endoscope channel associated with the present invention, the tool of cleaning the inside of an endoscope channel with a magnet or magnetic body and a non-magnetic tool to clean the channel inside of an endoscope may be provided. In this case, for example, the non-magnetic tool of cleaning the inside of an endoscope channel runs through the channel in a straight direction among branch channels and the tool of cleaning the inside of an endoscope channel with the magnet or magnetic body uses a magnetic field of the magnet or magnetic body configured outside the channel to be guided to the channel in a bending direction among branch channels to clean. Each tool of cleaning the inside of an endoscope channel can thus be guided to each branch channel to clean without varying a location and a magnetic force of the magnet or magnetic body configured outside the channel.

The system of cleaning the inside of an endoscope channel associated with the present invention comprises a feeding device, in which the above feeding device is provided with a feed section of a cleaning tool, a feed section of cleaning water, a connecting pipe and a switching section, in which preferably the above feed section of the cleaning tool is configured to accommodate a plurality of the above tools of cleaning the inside of an endoscope channel and provided with a vent of the cleaning tool to discharge one-by-one each tool of cleaning the inside of an endoscope channel along the above axial direction, the above feed section of cleaning water is provided with a feed port of cleaning water to feed cleaning water, the above connecting pipe is configured to connect one end thereof with a channel port of the above endoscope and the above switching section is connected with the above vent of the cleaning tool, the above feed port of cleaning water and other end of the above connecting pipe to receive one-by-one the above tool of cleaning the inside of an endoscope channel discharged from the above vent of the cleaning tool as well as feed it together with the above cleaning water fed from the above feed port of cleaning water from other end of the above connecting pipe to an inside of the above channel.

In this case, the feeding device can feed one-by-one the tool of cleaning the inside of an endoscope channel together with cleaning water to the channel inside, improving efficiency in cleaning operation. The switching section is preferably capable of automatically feeding the tool of cleaning the inside of an endoscope channel to the channel inside. The switching section is also preferably capable of configuring and varying a feed rate of the tool of cleaning the inside of an endoscope channel. In this case, optimum configuration can be chosen to improve a cleaning effect depending on kinds and a contamination level of the endoscope.

In the method of cleaning the inside of an endoscope channel related to the present invention, a granular tool of cleaning the inside of the endoscope channel with a diameter smaller than an inner diameter of the endoscope channel may be inserted through the above channel port of an endoscope into the inside of the above channel, followed by injecting cleaning water through the above channel port to move the above tool of cleaning the inside of the endoscope channel inside the above channel.

In the method of cleaning the inside of the endoscope channel related to the present invention, the granular tool of cleaning the inside of the endoscope channel, which moves inside the channel with cleaning water hits and contacts the inner wall of the channel, enabling to remove contaminants and the like attached to the inner wall surface of the channel and clean the inside of the channel.

The tool of cleaning the inside of an endoscope channel is preferably made from an inorganic substance readily soluble in an acid such as calcium carbonate, calcium phosphate and the like or foam thereof. In this case, acidic sterile water such as electrolytic acidic water and the like can be used as sterile cleaning water after cleaning to dissolve the tool of cleaning the inside of an endoscope channel in the sterile cleaning water. This allows dissolving the tool of cleaning the inside of an endoscope channel attached in the endoscope channel, exterior components and a cleaning vessel to prevent the tool of cleaning the inside of an endoscope channel from remaining therein.

The tool of cleaning the inside of an endoscope channel is also preferably composed of particles with a particle size of 18 to 70 meshes when the internal diameter of an endoscope channel is about 3 mm. When a particle size is larger than this value, particles could lead to overlap each other inside the channel to clog the inside of the channel. Also, when a particle size is smaller than this value, collision force due to hitting the inner wall of the channel to contact differs only slightly with frictional force due to a flow rate of cleaning water to reduce the efficiency of removing contaminants and the like.

The tool of cleaning the inside of an endoscope channel preferably has an apparent specific gravity in water of 1.1 to 2.5. When the specific gravity is equal to or smaller than the specific gravity of cleaning water, the tool of cleaning the inside of an endoscope channel, when discharged into the cleaning vessel, floats on a surface of cleaning water in a cleaning vessel to adhere to the external surface of an endoscope, resulting in formation of new contaminants. When the specific gravity is too large, the tool of cleaning the inside of an endoscope channel is poorly dispersed in the cleaning water injected into the inside of the channel to possibly cause uneven removal of contaminants in the inner wall of the channel and stagnation in the section with a slow flow rate to prevent cleaning.

The present invention can provide the system of cleaning the inside of an endoscope channel and the method of cleaning the inside of an endoscope channel to reduce a burden on an operator with enhanced safety with infection and contamination and a higher cleaning effect.

### BRIEF DESCRIPTION OF THE DRAWINGS

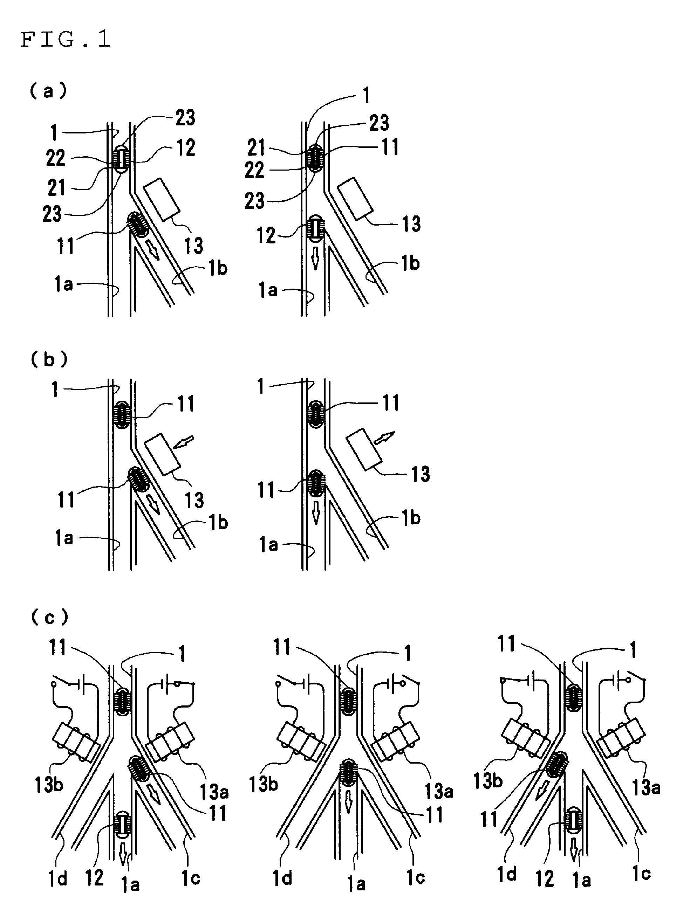

Figure 1 is a cross-sectional view of an embodiment of the present Example (a), a cross-sectional view of a modified Example (b) and a cross-sectional view of another modified Example (c) illustrating a tool of cleaning the inside of an endoscope channel, a system of cleaning the inside of a endoscope channel and a branching point of channels in a method of cleaning the inside of an endoscope channel.

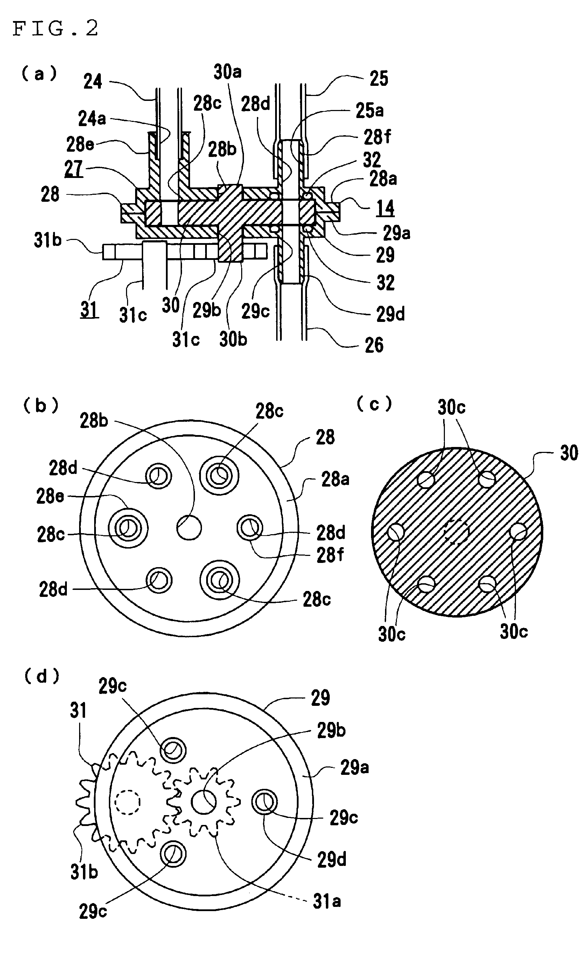

Figure 2 is a cross-sectional view illustrating a feeding device (a), a plan view illustrating an upper part of a housing of a switching section in the feeding device (b), a transverse cross-sectional view illustrating a sliding plate (c) and a plan view illustrating a lower part of a housing and a rotating mechanism, respectively, in the system of cleaning the inside of an endoscope channel shown in Figure 1.

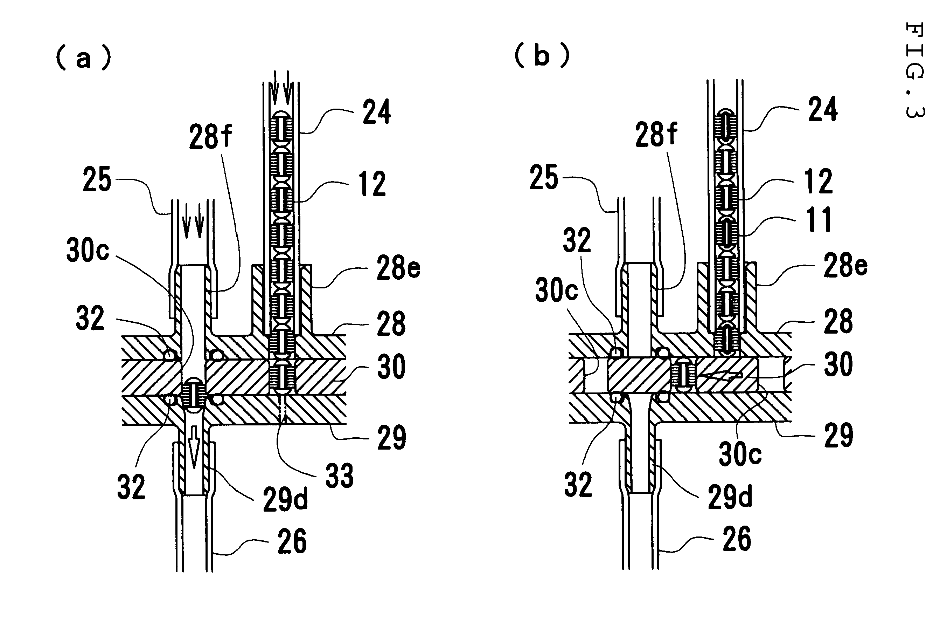

Figure 3 is a cross-sectional view illustrating a busy status of the feeding device in the system of cleaning the inside of an endoscope channel shown in Figure 1.

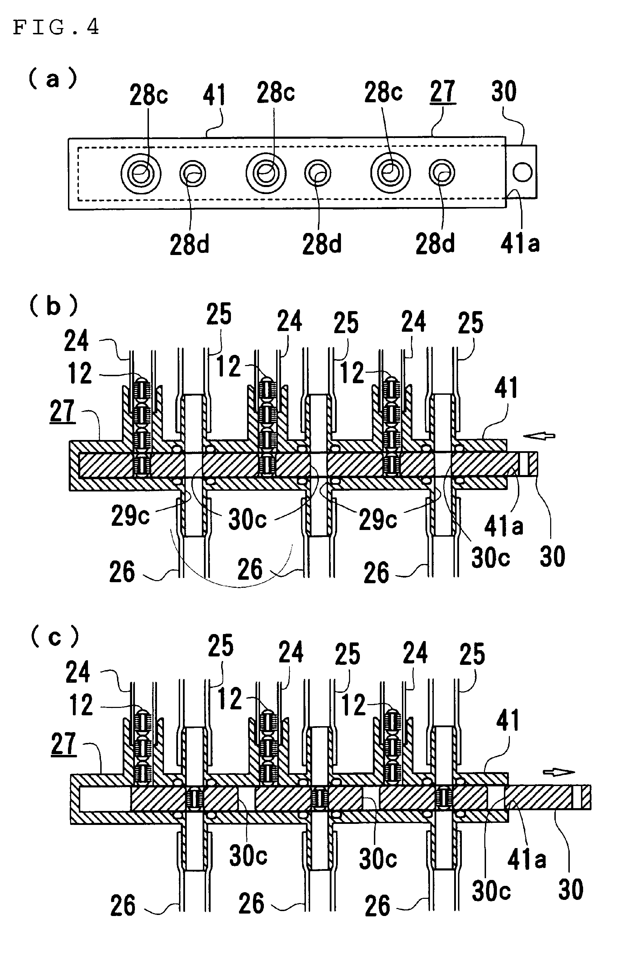

Figure 4 is a plan view of a modified Example of a feeding device (a), a cross-sectional view illustrating a busy status of pushing a slide plate thereof (b) and a cross-sectional view illustrating a busy status of pulling the slide plate thereof (c) in a system of cleaning the inside of an endoscope channel shown in Figure 1.

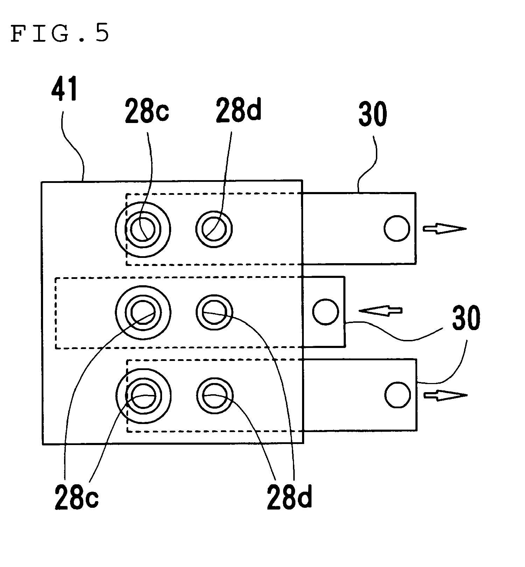

Figure 5 is a plan view illustrating another modified Example of a feeding device in a system of cleaning the inside of an endoscope channel shown in Figure 1.

### Explanation of Letters and Numerals

1: Channel
1a: Main channel
1b: Branch channel
11: First cleaning element
12: Second cleaning element
13: Guiding device
14: Feeding device
24: Feed section of cleaning tool
25: Feed section of cleaning water
26: Connecting pipe
27: Switching section
28: Upper part of housing
29: Lower part of housing
30: Slide plate
31: Rotating mechanism

### DETAILED DISCLOSURE OF THE PREFERRED EMBODIMENT

An embodiment of the present invention is described below based on the drawings.
Figures 1 to 5 illustrate a tool of cleaning the inside of an endoscope channel, a system of cleaning the inside of an endoscope channel and a method of cleaning the inside of an endoscope channel in the embodiment of the present invention.
As shown in Figures 1 and 2, the system of cleaning the inside of an endoscope channel comprises a first cleaning element 11, a second cleaning element 12, a guiding device 13 and a feeding device 14.
As shown in Figure 1(a), an endoscope branches a channel 1 into two of a main channel 1a in a straight direction and a branch channel 1b branched in a bending direction.

As shown in Figure 1, the first cleaning element 11 and the second cleaning element 12 are formed in an outer form of a cylindrical brush, in which many hairs 22 are planted on an external side surface of a cylindrical core material 21 perpendicularly to the side thereof. A core material 21 is made from a corrosion-resistant resin. Each hair 22 is flexible to be bent in a length direction and elastic in a direction perpendicular to the axis of the core material 21. The first cleaning element 11 and the second cleaning element 12 are equipped with a chip 23 with a smoothly curbed surface at both ends of the core material 21. Each chip 23 has a curbed surface with a curvature of a diameter smaller than an inner diameter of a channel 1 and is configured to face the curved surface thereof outwardly. The first cleaning element 11 and the second cleaning element 12 are formed such that an external diameter thereof is larger than an inner diameter of a channel 1 of an endoscope in a cross-section perpendicular to an axis of the core material 21 and a length thereof is longer than an inner diameter of the channel 1. The first cleaning element 11 and the second cleaning element 12 are formed in rotational symmetry with the axis of the core material 21 and provided with strength enough not to be compressed and deformed in a direction of the axis of the core material 21.

The first cleaning element 11 and the second cleaning element 12 form a tool of cleaning the inside of an endoscope channel. The first cleaning element 11 is provided with a magnetic body sealed and accommodated in the inside of the core material 21. The magnetic body is not magnetized. The second cleaning element 12 is not provided with a magnetic body and the like and non-magnetic. The first cleaning element 11 and the second cleaning element 12 are colored in different colors to be distinguished.

As shown in Figure 1(a), a guiding device 13 is provided with a magnet and configured outside a branching point of the channel 1. The guiding device 13 is configured on the side of a branch channel 1b to act on the magnetic body of the first cleaning element 11 and to guide the first cleaning element to the branch channel 1b among the channels 1.

As shown in Figure 2, a feeding device 14 comprises three feed sections of a cleaning tool 24, three feed sections of cleaning water 25, three connecting pipes 26 and a switching section 27. As shown in Figure 2(a), each feed section of the cleaning tool 24 is composed of a tubular cartridge and capable of accommodating a plurality of the first cleaning element 11 and the second cleaning element 12 along an axial direction. Each feed section of the cleaning tool 24 is provided with a vent of the cleaning tool 24a at one end such that the first cleaning element 11 and the second cleaning element 12 can be discharged one-by-one by gravitation in an axial direction from the vent of the cleaning tool 24a. Each feed section of cleaning water 25 is tubular and one end thereof is connected with a tank (not shown), which stores cleaning water. Each feed section of cleaning water 25 is provided with a feed port of cleaning water 25a at the other end, enabling to feed cleaning water from the feed port of cleaning water 25a. Each connecting pipe 26 can be connected through one end with a forceps channel port, a suction channel port and an air-supply and water-supply channel port of an endoscope.

As shown in Figure 2, a switching section 27 comprises an upper part of housing 28, a lower part of housing 29, a slide plate 30 and a rotating mechanism 31. As shown in Figure 2(b), the upper part of housing 28 is formed in a circular planar shape, in which a central part is formed in an upward convex shape and the periphery thereof is provided with a flange 28a. The upper part of housing 28 is provided with an axial hole 28b at the center location of the central part and three holes for an element 28c and three holes for cleaning water 28d alternately configured in intervals of 60 degree at the periphery thereof. Each hole for an element 28c is provided with a mounting section for an element 28e raised upward along the periphery thereof.

Each hole for cleaning water 28d is provided with a mounting section for cleaning 28f raised upward along the periphery thereof. As shown in Figure 2(d), the lower part of housing 29 is formed in a circular planar shape, in which a central part is formed in a downward convex shape and the periphery thereof is provided with a lower flange 29a. The lower part of housing 29 is provided with an axial hole 29b at the center location of the central part and three holes for an endoscope 29c in intervals of 120 degrees at the periphery thereof. Each hole for an endoscope 29c is provided with a mounting section for a connecting pipe 29d extended downward along the periphery thereof. The lower part of housing 29 is attached to a lower section of the upper part of housing 28 to match the lower flange 29a with the upper flange 28a such that each hole for an endoscope 29c is positioned right below each hole for cleaning water 28d. The upper part of housing 28 and the lower part of housing 29 are provided with a disk-like storage space between central parts of them.

As shown in Figure 2(c), a slide plate 30 is formed in a nearly disk-like shape. The slide plate 30 is provided with an upward protrusion 30a capable of inserting into the axial hole 28b of the upper part of housing 28 at a center location of a top surface and a downward protrusion 30b capable of inserting into the axial hole 29b of the lower part of housing 29 at a center location of a bottom surface. The slide plate 30 is provided with six through-holes in intervals of 60 degrees at the periphery of the center location. The slide plate 30 is accommodated in a 30c storage space between the upper part of housing 28 and the lower part of housing 29 by inserting the upward protrusion 30a into the axial hole 28b of the upper part of housing 28 and inserting the downward protrusion 30b into the axial hole 29b of the lower part of housing 29. The slide plate 30 can be rotated inside the storage space around the upward protrusion 30a and the downward protrusion 30b as a central axis and each through-hole 30c can be communicated with each hole for an element 28c, each hole for cleaning water 28d or each hole for an endoscope 29c in every rotation by 60 degrees. The downward protrusion 30b in the slide plate 30 is protruded below a bottom surface of the lower part of housing 29.

As shown in Figures 2(a) and 2(d), a rotating mechanism 31 is provided with a small gear 31a and a large gear 31b consisted of a spur gear. The small gear 31a is mounted by inserting the downward protrusion 30b beneath a center of the lower part of housing 29. The large gear 31b is engaged with the small gear 31 a and a drive shaft 31 c extended from a drive unit (not shown) is inserted at the center. The rotating mechanism 31 can rotate the small gear 31a and the slide plate 30 by rotating the large gear 31b by the drive unit.

As shown in Figures 2(a) and 3, in a switching section 27, the vent of a cleaning tool 24a in each feed section of a cleaning tool 24 is attached to each mounting section for an element 28e in the upper part of housing 28, a feed port of cleaning water 25a in each feed section of cleaning water 25 is attached to each mounting section of washing 28f and the other end of each connecting pipe 26 is attached to each mounting section of a connecting pipe 29d in the lower part of housing 29. The switching section 27 can accommodate the first cleaning element 11 or second cleaning element 12 discharged from the vent of cleaning tool 24a one-by-one in the inside of the through-hole 30c communicated with the vent of a cleaning tool 24a in every rotation of the slide plate 30 by 60 degrees. Furthermore, the switching section 27 can communicate the through-hole 30c accommodating the first cleaning element 11 or the second cleaning element 12 with a feed port of cleaning water 25a and the other end of the connecting pipe 26 in every rotation of the slide plate 30 by 60 degrees and feed the first cleaning element 11 or second cleaning element 12 together with cleaning water fed from the feed port of cleaning water 25a from the other end of the connecting pipe 26 to the inside of the channel 1.

As shown in Figure 2(a), a feeding device 14 is provided with an O-ring 32 around each hole for cleaning water in the upper part of housing 28 and each hole for an endoscope 29c in the lower part of housing 29 in order to prevent cleaning water from leaking between the upper part of housing 28, the lower part of housing 29 and the slide plate 30.

A method of cleaning the inside of an endoscope channel in the embodiment of the present invention is a method to be practiced using the tool of cleaning the inside of an endoscope channel and the system of cleaning the inside of an endoscope channel in the embodiment of the present invention. An endoscope for cleaning is at first accommodated in an inside of a cleaning vessel, in which cleaning water is pooled. One end of each connecting pipe 26 is connected one-by-one with a forceps channel port, a suction channel port and an air-supply and water-supply channel port of an endoscope. The feeding device 14 thereafter feeds the first cleaning element 11 and the second cleaning element 12 one-by-one to the inside of each channel 1 together with cleaning water such that an axial direction of the first cleaning element 11 or second cleaning element 12 conforms with a length direction of each channel 1.

At this time, the first cleaning element 11 and the second cleaning element 12 can be compressed in a direction perpendicular to the axis and be readily inserted into the inside of the channel 1, since they are provide with an external diameter larger than an inner diameter of the channel 1 in a cross-section perpendicular to the axis and elastic in a direction perpendicular to the axis. Insertion into an inside of the channel 1 may lead to a state of contacting the inner wall surface of the channel 1. A pressure of cleaning water is adjusted in this state to move the first cleaning element 11 and the second cleaning element 12 inside the channel 1.
The first cleaning element 11 and the second cleaning element 12 can thereby remove contaminant attached to the inner wall surface of the channel 1 to clean the inside of the channel 1.

As shown in Figure 1(a), when the first cleaning element 11 reaches a branching point of the channel 1, a magnet of the guiding device 13 acts on a magnetic body of the first cleaning element 11, enabling to guide the first cleaning element to clean a branch channel 1b among branch channels 1. When the second cleaning element 12 reaches a branching point of the channel 1, a magnet of the guiding device 13 does not act on the second cleaning element 12 because of non-magnetism of the second cleaning element 12, thus enabling the second cleaning element 12 to move through and clean a main channel 1a in a straight direction. Use of both the first cleaning element 11 and the second cleaning element 12 thus enables easy cleaning of the inside of the bifurcate channel 1.

A burden on an operator can be thus reduced as compared with careful cleaning with a brush, since cleaning is performed by inserting the first cleaning element 11 and the second cleaning element 12 into the inside of the channel 1, followed by injecting cleaning water. Safety with infection and contamination is enhanced since an operator does not directly contact contaminants. The first cleaning element 11 and the second cleaning element 12 passed through the inside of the channel 1 can be discharged into cleaning water in a cleaning vessel, since operation is performed after placing an endoscope in cleaning water in the cleaning vessel. This prevents droplets containing contaminants from splashing and re-contamination of the inside of the channel 1, leading to enhanced safety with infection and contamination and a higher cleaning effect.

When the first cleaning element 11 and the second cleaning element 12 are readily compressed to deform in an axial direction of the core material 21, they may be compressed in the axial direction by a water pressure conveyed when passing the area with higher passage resistance such as a joint of the channel 1 and a bend section, widening the diameter in a direction perpendicular to the axis depending on an amount compressed and thus increasing friction with the inner wall of the channel 1 to increase passage resistance and make passage difficult, potentially resulting in plugging the channel 1 depending on conditions. On the contrary, the first cleaning element 11 and the second cleaning element 12 are neither compressed nor deformed in an axial direction of the core material 21 so that they are not likely to make passage difficult through the channel 1 or plug it.

The first cleaning element 11 and the second cleaning element 12 are equipped with a chip 23 at both ends to prevent from entwining each other when disposed linearly. The first cleaning element 11 can be prevented from entwining together and adhering to deform in a step of manufacture and storage, since the magnetic body provided on the first cleaning element 11 is not magnetized. Use of a magnet permits selection of the first cleaning element 11 used and facilitate disposal of the first cleaning element 11.

Water pressure in cleaning water injected for cleaning may be switched between a positive pressure and a negative pressure to shuttle the first cleaning element 11 and the second cleaning element 12 inside the channel 1, increasing capability to clean contaminants. The tool of cleaning the inside of an endoscope channel is not limited to the first cleaning element 11 and the second cleaning element 12, but different kinds , combinations and surface nature of the material can be used to increase removal efficiency depending on a condition of contamination. The first cleaning element 11 and the second cleaning element 12 may be discarded after single use to separate the elements passed through the inside of the channel 1 from cleaning water discharged through a screen or grate mounted on a drain outlet of a cleaning vessel, thus readily being treated as a medical waste. The first cleaning element 11 and the second cleaning element 12 are preferably stored under sterilization until they are accommodated in each feed section of a cleaning tool 24 to be mounted on the switching section 27. Each feed section of a cleaning tool 24 may discharge the first cleaning element 11 and the second cleaning element 12 one-by-one by not gravity, but a spring, an air pressure, a pushrod and the like. As shown in Figure 3(a) with a case of an air pressure, a hole for air release 33 is preferably formed in a location of the lower part of housing 29 corresponding to each hole for an element 28c.

As shown in Figure 1(b), cleaning may be performed using only first cleaning element 11. In this case, a magnet of the guiding device 13 may be advanced near a branching point of the channel 1 to guide the first cleaning element 11 to the branch channel 1b and a magnet of the guiding device 13 may be distanced from a branching point of the channel 1 to guide the first cleaning element 11 to clean the main channel 1a.

As shown in Figure 1(c), the channel 1 of an endoscope may be branched to three of a main channel 1a in a straight direction, a first branch channel 1c and a second branch channel 1d and a guiding device 13 may be provided with a first electromagnet 13a configured on the side of the first branch channel 1c and a second electromagnet 13b configured on the side of the second branch channel 1d. In this case, an electric current is sent to only the first electromagnet 13a to guide the first cleaning element 11 to clean the first branch channel 1c, an electric current to the first electromagnet 13a and the second electromagnet 13b is turned off to guide the first cleaning element 11 to clean the main channel 1a and an electric current is sent to only the second electromagnet 13b to guide the first cleaning element 11 to clean the second branch channel 1d.

As shown in Figure 4, a switching section 27 of a feeding device 14 may be provided with an elongated rectangular solid housing 41, in which an upper part of housing 28 and a lower part of housing 29 are integrated, the housing 41 is provided with a rectangular storage space in an inside thereof, an opening 41 a of the storage space at an edge of one end, three holes for an element 28c and three holes for cleaning water 28d alternately on a top surface in a length direction and three holes for an endoscope 29c at a location of a bottom surface right below each hole for cleaning water 28d and a slide plate 30 is formed with an elongated rectangular plate and provided with six through-holes 30c along a length direction to be accommodated in a storage space slidable in a length direction to take in and out through the opening 41 a. As shown in Figures 4(b) and 4(c) with this case, the slide plate 30 is taken in and out through the opening 41 a to slide to accommodate the first cleaning element 11 or the second cleaning element 12 discharged from the vent of a cleaning tool 24a one-by-one in the through-hole 30c, allowing to feed the first cleaning element 11 or second cleaning element 12 accommodated together with cleaning water fed from the feed port of cleaning water 25a through the other end of the connecting pipe 26 to the inside of the channel 1. The slide plate 30 may be driven by a push-pull solenoid, a crank apparatus and the like.

As shown in Figure 5, the housing 41 may be further provided with each hole for an element 28c and each hole for cleaning water 28d aligning along a width direction respectively, and the slide plate 30 is composed of three parts, in which each may be provided with two through-holes 30c along a length direction and each through-hole 30c may be configured in lining in a width direction such that each through-hole 30 corresponds to each hole for an element 28c and each hole for cleaning water 28d. In this case, the first cleaning element 11 and the second cleaning element 12 can be separately fed to each channel. The number of the first cleaning element 11 and the second cleaning element 12 and a feeding timing thereof can be thus adjusted depending on a need of each channel.

## Claims

1. A system of cleaning the inside of an endoscope channel to clean the channel inside of an endoscope, comprising;
a tool of cleaning the inside of the endoscope channel and a guiding device,
wherein the endoscope branches the channel into a plurality of channels;
the tool of cleaning the inside of the endoscope channel is formed in a rotational symmetry with a given axis, provided with an external diameter larger than an inner diameter of the channel in a cross-section perpendicular to the axis at a given location, elastic in a direction perpendicular to the axis and provided with a magnet or magnetic body; and
the guiding device is provided with a magnet or magnetic body configured outside the channel to act on the magnet or magnetic body of the tool of cleaning the inside of an endoscope channel to guide the tool of cleaning the inside of an endoscope channel to one of the branch channels.

2. The system of cleaning the inside of an endoscope channel according to Claim 1 comprising;
a feeding device,
wherein the feeding device comprises a feed section of a cleaning tool, a feed section of cleaning water, a connecting pipe and a switching section, wherein the feed section of the inside of the cleaning tool is configured to accommodate a plurality of the tool of cleaning the endoscope channel and provided with a vent of the cleaning tool capable of discharging the tool of cleaning the inside of an endoscope channel one-by-one along the axial direction, the feed section of cleaning water is provided with a feed port of cleaning water capable of feeding cleaning water, the connecting pipe is configured to connect one end thereof with the channel port of an endoscope and the switching section is connected with the vent of the cleaning tool, the feed port of cleaning water and the other end of the connecting pipe to receive one-by-one the tool of cleaning the inside of an endoscope channel discharged from the vent of the cleaning tool, allowing to feed together with the cleaning water fed from the feed port of cleaning water from the other end of the connecting pipe to the inside of the channel.

3. A method of cleaning the inside of an endoscope channel to clean a channel inside of an endoscope,
wherein the endoscope branches the channel into a plurality of channels;
and the method comprises the steps of;
inserting a tool of cleaning the inside of an endoscope channel, which is formed in a rotational symmetry with a given axis, provided with an external diameter larger than an inner diameter of the endoscope channel in a cross-section perpendicular to the axis in a given location, elastic in a direction perpendicular to the axis and provided with a magnet or magnetic body through a channel port of the endoscope into the inside of the channel to conform the axial direction with a length direction of the channel;
injecting cleaning water from the channel port to move the tool of cleaning the inside of an endoscope channel inside the channel; and
acting a magnet or magnetic body configured outside the channel on the magnet or magnetic body of the tool of cleaning the inside of an endoscope channel to guide the tool of cleaning the inside of an endoscope channel to one of the branch channels.
